# EUROPEAN PATENT APPLICATION

(11) **EP 4 484 546 A1**
(43) Date of publication of application: **01.01.2025**
(21) Application number: 22928595.2
(22) Date of filing: 24.02.2022
(51) Int. Cl.: C12N 1/04, A01N 1/02

(54) **BIOLOGICAL SAMPLE PRESERVATIVE, PRODUCTION METHOD OF BIOLOGICAL SAMPLE PRESERVATIVE, PRESERVATION METHOD OF BIOLOGICAL SAMPLE, AND KIT FOR PRESERVING BIOLOGICAL SAMPLE**

(71) Applicant: Blue Industries Inc., Tokyo 130-0013 (JP)
(72) Inventor: KUJI Tomoaki, Tokyo 130-0013 (JP)
(74) Representative: Reddie & Grose LLP
(86) International application number: PCT/JP2022/007491
(87) International publication number: WO 2023/162065

(57) **Abstract**

[Problem] To provide a novel biological sample preservative that enables preservation of a biological sample at normal temperature, a production method of the biological sample preservative, a preservation method of a biological sample, and a kit for preserving a biological sample. [Solution] A biological sample preservative contains 1.0 to 10.0 mol/L of a substance having a polyatomic monovalent cation represented by a chemical formula NH4⁺ or a compound comprising the polyatomic monovalent cation. As the substance having the polyatomic monovalent cation represented by the chemical formula NH4⁺ or the compound comprising the polyatomic monovalent cation, for example, ammonium sulfate or ammonium nitrate can be used.

## Description

### Technical Field

The present disclosure relates to a biological sample preservative for preserving a biological sample, a production method of the biological sample preservative, a preservation method of biological sample, and a kit for preserving a biological sample.

### Background Art

Conventionally, an excellent preservation technique for various biological samples such as nucleic acids, cells, and tissues collected from organisms such as humans has been required in various industrial fields. However, such biological samples generally lose activity thereof gradually over time. Therefore, a technique of preserving the biological sample for a long period of time is a significantly important technique. For example, Patent Literature 1 describes a cryopreservation solution characterized by containing modified polyvinyl alcohol having an ethylene oxide group. Specifically, after cells or tissues of a living body are immersed in an equilibrium solution, the cells or the tissues are taken out together with a small amount of the equilibrium solution using a tubular jig such as a pipette and are moved into the cryopreservation solution. Then, after the cells or the tissues are immersed in the cryopreservation solution for a predetermined time, the cells or the tissues are taken out together with a small amount of the cryopreservation solution, and the cells or the tissues are attached dropwise together with a small amount of the cryopreservation solution onto a sheet of a cryopreservation jig. Thereafter, the sheet on which the cells or the tissues are placed is immersed in a cooling medium such as liquid nitrogen and is frozen. Through the above steps, the cells and the tissues of the living body are preserved through the preservation technique described in Patent Literature 1.

However, in the preservation technique described in Patent Literature 1, since the cryopreservation solution is used for preservation of the cells and the tissues of the living body, not only various steps are included until preservation thereof, but also it is necessary to maintain a frozen environment after preservation, which results in a complicated handling step.

### Citation List

### Patent Literature

Patent Literature 1: JP 2020-39261 A

### Summary of Invention

### Technical Problem

Therefore, an object of the present disclosure is to provide a novel biological sample preservative that enables preservation of a biological sample at normal temperature, a production method of the biological sample preservative, a preservation method of a biological sample, and a kit for preserving a biological sample.

### Solution to Problem

In order to solve the above problems, the present diclosers have found a biological sample preservative capable of preserving a biological sample at normal temperature by containing 1.0 to 10.0 mol/L of a substance having a polyatomic monovalent cation represented by a chemical formula NH4⁺ or a compound comprising the polyatomic monovalent cation. Therefore, a first phase of the present disclosure is (1) a biological sample preservative including 1.0 to 10.0 mol/L of a substance having a polyatomic monovalent cation represented by a chemical formula NH4⁺ or a compound comprising the polyatomic monovalent cation.

A preferred aspect of the present disclosure is (2) the biological sample preservative according to (1), in which a concentration of the substance having the polyatomic monovalent cation or the compound comprising the polyatomic monovalent cation is 1.7 to 5.0 mol/L.

A preferred aspect of the present disclosure is (3) the biological sample preservative according to (1) or (2), in which the substance having the polyatomic monovalent cation or the compound comprising the polyatomic monovalent cation contains at least one of ammonium sulfate and ammonium nitrate.

A preferred aspect of the present disclosure is (4) the biological sample preservative according to any one of (1) to (3), further including 0.004 to 10.000 mol/L of a chelating reagent.

A preferred aspect of the present disclosure is (5) the biological sample preservative according to (4), in which a concentration of the chelating reagent is 0.006 to 0.500 mol/L.

A preferred aspect of the present disclosure is (6) the biological sample preservative according to (4), in which the chelating reagent contains at least one of sodium citrate and trisodium citrate monohydrate.

A preferred aspect of the present disclosure is (7) the biological sample preservative according to any one of (1) to (6), further including 0.004 to 10.000 mol/L of a buffer.

A preferred aspect of the present disclosure is (8) the biological sample preservative according to (7), in which a concentration of the buffer is 0.010 to 5.000 mol/L.

A preferred aspect of the present disclosure is (9) the biological sample preservative according to (7) or (8), in which the buffer is ethylenediaminetetraacetic acid.

A preferred aspect of the present disclosure is (10) the biological sample preservative according to any one of (1) to (9), further including 0.25 to 30 wt% of an excipient.

A preferred aspect of the present disclosure is (11) the biological sample preservative according to (1) to (10), in which the biological sample preservative is a freeze-dried powder.

Further, in order to solve the above problems, the present disclosers have found a production method of a biological sample preservative capable of preserving a biological sample at normal temperature by containing 1.0 to 10.0 mol/L of a substance having a polyatomic monovalent cation represented by a chemical formula NH4⁺ or a compound comprising the polyatomic monovalent cation. Therefore, another phase of the present disclosure is (12) a production method of a biological sample preservative, the method including a first addition step of adding, to a predetermined solvent, 1.0 to 10.0 mol/L of a substance having a polyatomic monovalent cation represented by a chemical formula NH4⁺ or a compound comprising the polyatomic monovalent cation.

A preferred aspect of the present disclosure is (13) the production method of the biological sample preservative according to (12), further including a second addition step of further adding 0.25 to 30 wt% of an excipient to a solution obtained by the first addition step.

A preferred aspect of the present disclosure is (14) the production method of a biological sample preservative according to (13), further including a freeze-drying step of freeze-drying, at -10°C to -100°C, a solution obtained by the second addition.

A preferred aspect of the present disclosure is (15) the production method of a biological sample preservative according to (12), further including a dehydration step of dehydrating, under a heated condition or a vacuum condition, a solution obtained by the first addition step.

In order to solve the above problems, the present disclosers have found a preservation method of a biological sample using a biological sample preservative solution containing 1.0 to 10.0 mol/L of a substance having a polyatomic monovalent cation represented by a chemical formula NH4⁺ or a compound comprising the polyatomic monovalent cation. Therefore, another phase of the present disclosure includes (16) a preservation step of immersing, to be preserved, a biological sample in a biological sample preservative solution containing 1.0 to 10.0 mol/L of a substance having a polyatomic monovalent cation represented by a chemical formula NH4⁺ or a compound comprising the polyatomic monovalent cation.

Additionally, in order to solve the above problems, the present disclosers have found a kit for preserving a biological sample, in which the kit includes a biological sample preservative containing 1.0 to 10.0 mol/L of a substance having a polyatomic monovalent cation represented by a chemical formula NH4⁺ or a compound comprising the polyatomic monovalent cation. Therefore, another phase of the present disclosure is (17) a kit for preserving a biological sample, the kit including a biological sample preservative containing 1.0 to 10.0 mol/L of a substance having a polyatomic monovalent cation represented by a chemical formula NH4⁺ or a compound comprising the polyatomic monovalent cation.

### Advantageous Effects of Invention

The present disclosure can provide a novel biological sample preservative that enables preservation of a biological sample at normal temperature, a production method of the biological sample preservative, a preservation method of a biological sample, and a kit for preserving a biological sample.

### Brief Description of Drawings

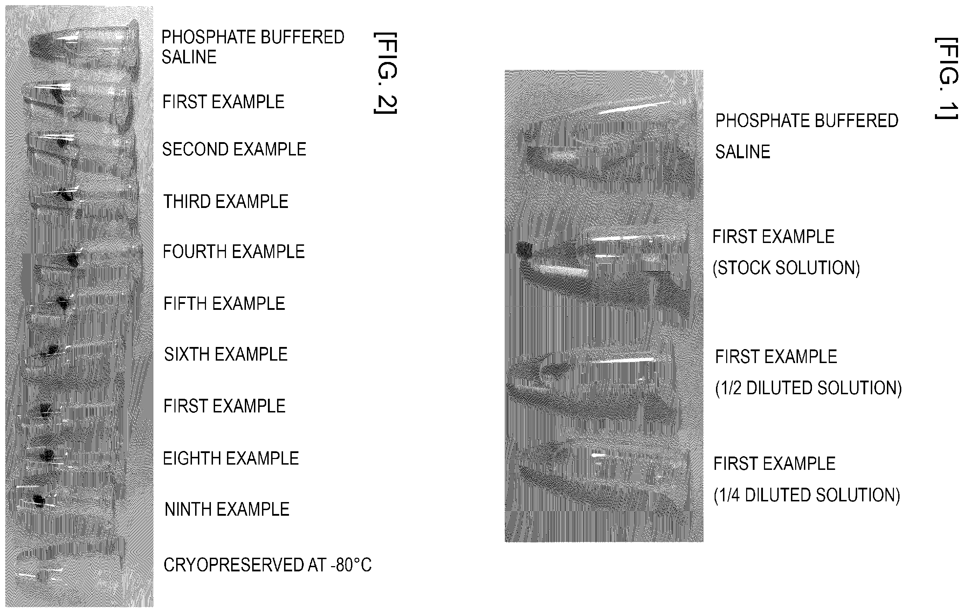

Fig. 1 is a diagram illustrating an image of each microtube after each of the mouse livers is preserved in a corresponding one of: a stock solution of a biological sample preservative of a first example; a 1/2 diluted solution thereof; a 1/4 diluted solution thereof; and a phosphate buffered saline prepared as a negative control, at 37°C for 24 hours.
Fig. 2 is a diagram illustrating an image of each microtube after each of the mouse livers is preserved in a corresponding one of the biological sample preservatives of the first to ninth examples and phosphate buffered saline prepared as a negative control, at 37°C for 24 hours.

### Description of Embodiments

Hereinafter, a mode for implementing a biological sample preservative, a production method of the biological sample preservative, and a preservation method of a biological sample of the present disclosure will be described in detail. However, the following embodiments are examples for describing the present disclosure, and the present disclosure is not limited only to the embodiments.

### <Production of Biological Sample Preservative>

In the present disclosure, the biological sample preservative can be produced through, for example, a step of adding each component to a predetermined solvent, and a freeze-drying step or a dehydration step as necessary. Hereinafter, a production step of the biological sample preservative will be specifically described.

Here, in the present disclosure, a biological sample is a sample collected from a living body or used for the living body, and is an organ, a tissue, a cell (including a cultured cell), a body fluid, a nucleic acid, a protein, or a combination thereof. More preferably, a nucleic acid, a protein, or a combination thereof is used as the biological sample. It is noted that these biological samples may include a sample artificially synthesized for a living body in addition to the sample collected from the living body. Naturally, the invention according to the present disclosure is not limited only to those exemplified herein.

In addition, the biological sample preservative to be produced is preferably provided in the form of a solution when preserving a living body, but may be in any form such as a dry form, a solid form, a powder form, an anhydrous form, or a granular form. When the biological sample preservative is not in the form of a solution, the biological sample preservative is preferably used after being dissolved in a predetermined solvent so as to have a desired concentration thereof. In addition, the invention according to the present disclosure is not limited only to these forms.

Further, the biological sample preservative to be produced may be provided as a preservative or a preservative composition having the above-described form, or may be provided as a kit for preserving a biological sample including, for example, a container for biological sample preservation and a biological sample collection tool, in addition to the biological sample preservative.

### 1. First Addition Step

In the present disclosure, in a first addition step, first, a predetermined solvent is put into a necessary amount container and is stirred at normal temperature of 5 to 50°C, more preferably 10 to 40°C. Although pure water is typically used as the solvent, any of treated water such as: filtered water treated by filtration, distillation, deionization, or a combination thereof; purified water; distilled water; deionized water; or a combination thereof, and water containing any of these treated water may be used.

Thereafter, a buffer is added to the stirred solvent at a predetermined concentration. Various buffers such as a phosphate buffer solution, a tris hydrochloride acid buffer solution, an ethylenediaminetetraacetic acid buffer solution, a tris-ethylenediaminetetraacetic acid buffer solution, a tris-acetate-ethylenediaminetetraacetic acid buffer solution, a tris-boric acidethylenediaminetetraacetic acid buffer solution, an SSC buffer solution, an SSPE buffer solution, a sodium citrate buffer solution, a carbonate-bicarbonate buffer solution, a sodium borate buffer solution, a 2-morpholinoethanesulfonic acid buffer solution, and combinations thereof can be used as the buffer to be added. Among the buffers, an ethylenediaminetetraacetic acid buffer solution is more preferably used as the buffer. However, the invention according to the present disclosure is not limited only to these buffer solutions. In addition, the buffer solution is added so as to have a concentration of 0.004 to 10.000 mol/L, more preferably 0.010 to 5.000 mol/L in the biological sample preservative to be finally used.

Next, materials such as other main components and additive materials are added to the solution to which the buffer solution has been added. The main component includes a substance having a polyatomic monovalent cation represented by the chemical formula NH4⁺ or a compound comprising the polyatomic monovalent cation. Such substances or compounds are selected from a group consisting of ammonium sulfate, ammonium nitrate, and combinations thereof. By adding the substance having the polyatomic monovalent cation represented by NH4⁺ or the compound comprising the polyatomic monovalent cation, that is, a substance or a compound containing an ammonium ion, it is possible to further enhance a preservation effect and preservation stability of a biological sample or the like at normal temperature. It is noted that the invention according to the present disclosure is not limited only to these buffer solutions.

The substance having the polyatomic monovalent cation represented by the chemical formula NH4⁺ or the compound comprising the polyatomic monovalent cation is added so as to have a concentration of 1.0 to 10.0 mol/L, more preferably 1.7 to 5.0 mol/L in the biological sample preservative to be finally used.

Other components can include a chelating reagent. As such a chelating reagent, various chelating reagents such as sodium citrate, trisodium citrate monohydrate, sodium borate, sodium fluoride, ethylenediaminetetraacetic acid, or a combination thereof can be used. By adding these chelating reagents, it is possible to suppress action of the nucleolytic enzyme and to further enhance preservation stability of a biological sample such as a nucleic acid at normal temperature. It is noted that the invention according to the present disclosure is not limited only to these chelating reagents. The chelating reagent is added so as to have a concentration of 0.004 to 10.000 mol/L, more preferably 0.006 to 0.500 mol/L in the biological sample preservative to be finally used.

Other components can include stabilizers. As such stabilizers, it is possible to use various materials such as 4-(2-aminoethyl)benzenesulfonyl fluoride hydrochloride, aprotinin, E-64, leupeptin hemisulfate monohydrate, silicon carbide, 4-hydroxybenzoic acid, N, N-dialkylpropanamide, 3-morpholinopropanesulfonic acid, polyethylene glycol, Polyoxyethylene (poly(oxyethylene)) polymer, polyoxyetylene (poly(oxyetylene) polymer, mono-ethylene glycol(1,2-ethanediol), glutathione, lithium salt, chaotropic agent, guanidinium salt, guanidine hydrochloride, urea, polyamines, biuret, arginine, cosmotropes, polyethylene glycol, polyvinylpyrrolidone, arginine ethyl ester, glycine, amino acid alkyl ester, amino acid amide, a compound having a guanidinium group, or a combination thereof. By adding these stabilizers, it is possible to stabilize biological samples such as cells, proteins, and nucleic acids. Furthermore, among these stabilizers, 4-(2-aminoethyl)benzenesulfonyl fluoride hydrochloride, aprotinin, E-64, leupeptin hemisulfate monohydrate, or a combination thereof can particularly enhance stability by inhibiting protease activity. The invention according to the present disclosure is not limited only to these stabilizers. An amount of the stabilizer to be added may be appropriately adjusted depending on a stabilizer selected, but when 4-(2-aminoethyl)benzenesulfonyl fluoride hydrochloride, aprotinin, E-64, leupeptin hemisulfate monohydrate, or a combination thereof is selected, the stabilizer can be added at a concentration of 0.001 to 1.0 wt%, more preferably 0.01 to 0.10 wt%.

As other components, a sterilizing/antibacterial agent can be included. As such a sterilizing/antibacterial agent, various substances such as amoxicillin trihydrate, cefotaxime sodium, clarithromycin, erythromycin, pyrethrin, metofluthrin, transfluthrin, sodium azide, 4-hydroxybenzoic acid, alcohol, a surfactant, or a combination thereof can be used. It is noted that the invention according to the present disclosure is not limited only to these sterilizing/antibacterial agents. An amount of the sterilizing/antibacterial agent to be added can be appropriately adjusted depending on a stabilizer selected, but the sterilizing/antibacterial agent can be added at a concentration of 0.001 to 1.0 wt%, more preferably 0.01 to 0.10 wt%.

Although not specifically described in detail, other additives such as a surfactant, a pH adjusting agent, and a colorant may be appropriately included in addition to the above-described components.

### 2. Stirring Step

As described above, the solution to which each component is added in the first addition step is stirred at normal temperature of 5 to 50°C, more preferably 10 to 40°C, in a necessary amount container. The stirring is performed until the added components are completely dissolved therein. Then, the biological sample preservative according to the present disclosure is obtained as a solution by completely dissolving the added components. As described above, by producing the biological sample preservative containing the substance having the polyatomic monovalent cation represented by the chemical formula NH4⁺ or the compound comprising the polyatomic monovalent cation, a biological sample preservative having more excellent preservation stability can be obtained.

### 3. Freeze-Drying Step

The biological sample preservative produced as described above can be further processed into a given solid form such as a dry form, a solid form, a powder form, an anhydrous form, or a granular form in consideration of convenience of preservation and transportation of the biological sample preservative itself. Then, by treating the biological sample preservative (solution) produced as described above in the freeze-drying step, it is possible to obtain the biological sample preservative in these forms. It is noted that the freeze-drying step is not an essential step and can be performed as desired.

### 3-1. Second Addition Step

First, in the freeze-drying step, as a second addition step, an excipient is added to the biological sample preservative (solution) produced as described above and is dissolved by stirring. As such an excipient, a polyol, a monosaccharide, a disaccharide, a polysaccharide, an amino acid, a peptide, a protein, a hydrophilic polymer, or a combination thereof is used. Among these excipients, as an excipient, it is preferable to use lactose, glucose, sucrose, crystalline cellulose, calcium sulfate, calcium carbonate, talc, titanium oxide, erythritol, mannitol, sorbitol, trehalose, sucrose, methyl cellulose, hydroxypropyl cellulose, hydroxypropyl methyl cellulose, carmellose sodium, pullulan, dextrin, gum arabic, agar, gelatin, tragacanth, sodium alginate, polyvinylpyrrolidone, polyvinyl alcohol, or a combination thereof, and particularly, it is possible to preferably use calcium carbonate, dextrin, or a combination thereof. It is noted that the invention according to the present disclosure is not limited only to these excipients.

An added amount of these excipients may be appropriately adjusted depending on an excipient selected, but it is possible to add these excipients at a concentration of 0.25 to 30 wt%, more preferably 0.1 to 10.0 wt%.

Here, the biological sample preservative produced as described above contains the substance having the polyatomic monovalent cation represented by the chemical formula NH4⁺ or the compound comprising the polyatomic monovalent cation, typically ammonium sulfate or ammonium nitrate. Therefore, when the biological sample preservative is dehydrated and is frozen in the freeze-drying step, a freezing point is rapidly reached, so that ammonium sulfate or the like may explode in a freeze-drying device. In the present disclosure, this explosion can be prevented by adding a predetermined amount of the excipient in advance in the freeze-drying step as described above.

### 3-2. Freeze-Drying Step

The excipient is dissolved in the biological sample preservative, then, the solution in which the excipient is dissolved is subjected to freeze-drying treatment in the freeze-drying step. Although various methods can be used for this freeze-drying treatment, as an example, the solution in which the excipient is dissolved is degassed under vacuum conditions for a predetermined time (for example, 60 minutes), and then is left under a predetermined temperature (for example, -75°C) conditions for a predetermined time (for example, 60 minutes). Thereafter, the obtained biological sample preservative slowly returns to normal temperature and normal pressure, thereby obtaining a freeze-dried biological sample preservative. It is noted that, through the freeze-drying step or through a tableting step or a granulation step after the freeze-drying step, the biological sample preservative can be processed into a desired form.

### 4. Dehydration Step

It is also possible to process the biological sample preservative, produced by the first addition step and the stirring step, into a given solid shape such as a dry form, a solid form, a powder form, an anhydrous form, or a granular form in consideration of convenience of preservation and transportation of the biological sample preservative itself. Then, the biological sample preservative (solution) produced as described above is subjected to a dehydration step, whereby the biological sample preservative in these forms can be obtained. It is noted that the dehydration step is not an essential step, and can be performed as desired.

The necessary amount container containing the biological sample preservative (solution) produced by the above steps is put into a vacuum device and is left for a predetermined time (such as 24 hours). Thereafter, the obtained biological sample preservative slowly returns to normal pressure, thereby obtaining a dehydrated biological sample preservative. It is noted that, through the tableting step or the granulation step after the dehydration step, the dehydrated biological sample preservative can be processed into a desired form.

As described above, it is possible to produce a desired biological sample preservative through the first addition step and the stirring step, and as needed, the freeze-drying step or the dehydration step.

### <Constitution of Biological Sample Preservative>

The biological sample preservative produced through the above-described production steps contains the following components. That is, the biological sample preservative contains, at least, 1.0 to 10.0 mol/L, more preferably 1.7 to 5.0 mol/L of "a substance having a polyatomic monovalent cation represented by the chemical formula NH4⁺ or a compound comprising the polyatomic monovalent cation" in a predetermined solvent. Furthermore, the biological sample preservative may contain, as desired: 0.004 to 10.000 mol/L, more preferably 0.010 to 5.000 mol/L of a "buffer"; 0.004 to 10.000 mol/L, more preferably 0.006 to 0.500 mol/L of a "chelating reagent"; 0.001 to 1.0 wt%, more preferably 0.01 to 0.10 wt% of a "stabilizer"; and 0.001 to 1.0 wt%, more preferably 0.01 to 0.10 wt% of a "sterilizing/antibacterial agent". In addition, the biological sample preservative may contain 0.25 to 30 wt%, more preferably 0.1 to 10.0 wt% of an "excipient".

Further, although not specifically described herein, the biological sample preservative produced through the above production steps is adjusted to have a pH of 4.0 to 10.0, more preferably 6.0 to 8.0 by adding a pH adjusting agent or the like. This makes it possible to further enhance preservation stability of the biological sample.

It is noted that, although not specifically described herein, in addition to each component described above, other additives such as a surfactant, a pH adjusting agent, a colorant, etc., may be appropriately contained.

### <Preservation Method of Biological Sample>

In a preservation method using the biological preservative, for example, the biological sample preservative is used to preserve an organ, a tissue, a cell (including a cultured cell), a body fluid, a nucleic acid, a protein, or a combination thereof at normal temperature. Specifically, the biological sample preservative produced in the first addition step and the stirring step is dispensed into a predetermined container (for example, a vial bottle, a microtube, or the like). Then, the biological sample such as an organ, a tissue, a cell (including a cultured cell), a body fluid, a nucleic acid, a protein, or a combination thereof collected from a living body is immersed in the container and is left at normal temperature (for example, 5 to 50°C, more preferably 10 to 40°C). This makes it possible to preserve the collected biological sample at normal temperature.

When the biological sample preservative has been processed into a form such as a dry form, a solid form, a powder form, an anhydrous form, or a granular form by the freeze-drying step or the dehydration step, the biological sample preservative can be dissolved in a predetermined solvent so that each component has a concentration of a biological sample preservative in a solution state and then can be used (the concentration may be appropriately adjusted depending on the amount of the solvent). It is noted that, although pure water is typically used as the solvent, any of treated water such as: filtered water treated by filtration, distillation, deionization, or a combination thereof; purified water; distilled water; deionized water; or a combination thereof, and water containing any of these treated water may be used.

As described above, according to the present disclosure, it is possible to provide a novel biological sample preservative capable of preserving a biological sample at normal temperature, a production method of the biological sample preservative, and a preservation method of a biological sample.

### Examples

Hereinafter, the present disclosure will be described in more detail with reference to examples, but the present disclosure is not limited thereto.

### <Production of Biological Sample Preservative>

Pure water as a solvent was put in a heat-resistant bottle having a predetermined volume (2L glass medium bottle (manufactured by Thermo Fisher Scientific K.K.)), and after a stirrer with temperature control (product name: Hot Stirrer REXIM RSH-4DN (manufactured by AS ONE Corporation)) was set to 37°C, the pure water was stirred at a rotation speed of 60 rpm. Thereafter, a buffer solution as described in the following Tables 1 to 3 was added to the pure water and was further stirred until the buffer solution was completely dissolved. Next, ammonium sulfate and other components described in the following Tables 1 to 3 were added at concentrations described in the following Table 1, and were stirred until the respective components were completely dissolved, and each of the biological sample preservatives (first to ninth examples) was produced as a solution.

**[Table 1]**

| | Component | Concentration |
|---|---|---|
| | Pure water | - |
| | Ammonium sulfate | 3.531 mol/L |
| | (Chelating reagent) 1M Sodium citrate | 0.013 mol/L |
| | (Buffer) 0.5M Ethylenediaminetetraacetic acid buffer solution | 0.013 mol/L |
| | (pH adjusting agent) Sulfuric acid | trace amount |
| First example | (Stabilizer) 4-(2-Aminoethyl)benzenesulfonyl fluoride hydrochloride (AEBSF) | 1 wt% |
| | (Stabilizer) Aprotinin | 0.010 wt% |
| | (Stabilizer) E-64 | 0.015 wt% |
| | (Stabilizer) Leupeptin hemisulfate monohydrate | 0.010 wt% |
| | Pure water | |
| | Ammonium sulfate | 3.531 mol/L |
| | (Chelating reagent) 1M Sodium citrate | 0.013 mol/L |
| | (Buffer) 0.5M Ethylenediaminetetraacetic acid buffer solution | 0.030 mol/L |
| Second example | (pH adjusting agent) Sulfuric acid | trace amount |
| | (Sterilizing/antibacterial agent) Amoxicillin trihydrate | 0.05 wt% |
| | (Sterilizing/antibacterial agent) Cefotaxime sodium | 0.05 wt% |
| | (Sterilizing/antibacterial agent) Clarithromycin | 0.05 wt% |
| | (Sterilizing/antibacterial agent) Erythromycin | 0.05 wt% |
| Third example | Pure water | - |
| | Ammonium sulfate | 3.531 mol/L |
| | (Chelating reagent) 1M Sodium citrate | 0.013 mol/L |
| | (Buffer) 0.5M Ethylenediaminetetraacetic acid buffer solution | 0.030 mol/L |
| | (pH adjusting agent) Sulfuric acid | trace amount |
| | (Chelating reagent) Sodium borate | 3 wt% |
| | (Sterilizing/antibacterial agent) Pyrethrin | 0.05 wt% |
| | (Sterilizing/antibacterial agent) Metofluthrin | 0.05 wt% |
| | (Sterilizing/antibacterial agent) Transfluthrin | 0.05 wt% |

**[Table 2]**

| | Component | Concentration |
|---|---|---|
| | Pure water | - |
| | Ammonium sulfate | 4.000 mol/L |
| | (Buffer) 0.5M Ethylenediaminetetraacetic acid buffer solution | 0.010 mol/L |
| | (Buffer) 2-Morpholinoethanesulfonic acid | 0.100 mol/L |
| Fourth example | (pH adjusting agent) Sulfuric acid | trace amount |
| | (Stabilizer) 4-(2-Aminoethyl)benzenesulfonyl fluoride hydrochloride (AEBSF) | 0.05 wt% |
| | (Stabilizer) Aprotinin | 0.05 wt% |
| | (Stabilizer) E-64 | 0.05 wt% |
| | (Stabilizer) Leupeptin hemisulfate monohydrate | 0.05 wt% |
| | Pure water | |
| | Ammonium sulfate | 4.000 mol/L |
| | (Buffer) 0.5M Ethylenediaminetetraacetic acid buffer solution | 0.010 mol/L |
| | (Buffer) 2-Morpholinoethanesulfonic acid | 0.100 mol/L |
| Fifth example | (pH adjusting agent) Sulfuric acid | trace amount |
| | (Sterilizing/antibacterial agent) Amoxicillin trihydrate | 0.05 wt% |
| | (Sterilizing/antibacterial agent) Cefotaxime sodium | 0.05 wt% |
| | (Sterilizing/antibacterial agent) Clarithromycin | 0.05 wt% |
| | (Sterilizing/antibacterial agent) Erythromycin | 0.05 wt% |
| | Pure water | - |
| | Ammonium sulfate | 4.000 mol/L |
| | (Buffer) 0.5M Ethylenediaminetetraacetic acid buffer solution | 0.010 mol/L |
| | (Buffer) 2-Morpholinoethanesulfonic acid | 0.100 mol/L |
| Sixth example | (pH adjusting agent) Sulfuric acid | trace amount |
| | (Chelating reagent) Sodium borate | 3 wt% |
| | (Sterilizing/antibacterial agent) Pyrethrin | 0.05 wt% |
| | (Sterilizing/antibacterial agent) Metofluthrin | 0.05 wt% |
| | (Sterilizing/antibacterial agent) Transfluthrin | 0.05 wt% |

**[Table 3]**

| | Component | Concentration |
|---|---|---|
| | Pure water | - |
| | Ammonium sulfate | 5.297 mol/L |
| | (Chelating reagent) 1M Trisodium citrate monohydrate | 0.025 mol/L |
| | (Buffer) 0.5M Ethylenediaminetetraacetic acid buffer solution | 0.020 mol/L |
| Seventh example | (pH adjusting agent) Sulfuric acid | trace amount |
| | (Stabilizer) 4-(2-Aminoethyl)benzenesulfonyl fluoride hydrochloride (AEBSF) | 1 wt% |
| | (Stabilizer) Aprotinin | 0.010 wt% |
| | (Stabilizer) E-64 | 0.015 wt% |
| | (Stabilizer) Leupeptin hemisulfate monohydrate | 0.010 wt% |
| | Pure water | - |
| | Ammonium sulfate | 5.297 mol/L |
| | (Chelating reagent) 1M Trisodium citrate monohydrate | 0.025 mol/L |
| | (Buffer) 0.5M Ethylenediaminetetraacetic acid buffer solution | 0.020 mol/L |
| Eighth example | (pH adjusting agent) Sulfuric acid | trace amount |
| | (Sterilizing/antibacterial agent) Amoxicillin trihydrate | 0.05 wt% |
| | (Sterilizing/antibacterial agent) Cefotaxime sodium | 0.05 wt% |
| | (Sterilizing/antibacterial agent) Clarithromycin | 0.05 wt% |
| | (Sterilizing/antibacterial agent) Erythromycin | 0.05 wt% |
| | Pure water | - |
| | Ammonium sulfate | 5.297 mol/L |
| | (Chelating reagent) 1M Trisodium citrate monohydrate | 0.025 mol/L |
| | (Buffer) 0.5M Ethylenediaminetetraacetic acid buffer solution | 0.020 mol/L |
| Ninth example | (pH adjusting agent) Sulfuric acid | trace amount |
| | (Chelating reagent) Sodium borate | 3 wt% |
| | (Sterilizing/antibacterial agent) Pyrethrin | 0.05 wt% |
| | (Sterilizing/antibacterial agent) Metofluthrin | 0.05 wt% |
| | (Sterilizing/antibacterial agent) Transfluthrin | 0.05 wt% |

### <Measurement of Preservation Effect of Biological Sample>

A biological sample preservation effect of each of the biological sample preservatives of the first to ninth examples produced as described above was measured as follows.

### [First Experiment]

The biological sample preservative of the first example was taken from each heat-resistant bottle, and 1 mL of each of a stock solution, a 1/2 diluted solution, and a 1/4 diluted solution was dispensed into a corresponding one of 1.5 mL microtubes. Then, 1.3 mg of a mouse liver was collected as a biological sample, was put into the microtubes into which the respective biological sample preservative was dispensed, and was immersed in each of the biological sample preservatives. At this time, as a negative control, 1 mL of phosphate buffered saline was dispensed into a 1.5 mL microtube, and similarly, 1.3 mg of the mouse liver was immersed in the phosphate buffered saline. Then, each of the biological samples put into a corresponding one of the microtubes was left to stand and was preserved under normal pressure at 37°C for 24 hours.

After a lapse of 24 hours, each of the livers each serving as a biological sample was taken out from a corresponding one of the microtubes, and RNA extraction was performed on each of the livers. By using a commercially available RNA extraction kit (product name: RNeasy Plus Mini Kit (manufactured by QIAGEN)), RNA extraction was performed by a method specified with the kit. Then, preservation stability of the biological sample was evaluated for each of the biological samples put into a corresponding one of the microtubes by measuring the concentration of RNA obtained from each of the biological samples. The concentration of RNA was measured by an electrophoresis system device (product name: Bioanalyzer 2100 (manufactured by Agilent)) in accordance with a method designated with the electrophoresis system device.

Fig. 1 is a diagram illustrating an image of each microtube after each of the mouse livers is preserved in a corresponding one of: a stock solution of the biological sample preservative of the first example; a 1/2 diluted solution thereof; a 1/4 diluted solution thereof; and a phosphate buffered saline prepared as a negative control, at 37°C for 24 hours. Further, Table 4 shows concentrations of RNA obtained from the mouse livers respectively preserved in: the stock solutions of the biological sample preservative of the first example; the 1/2 diluted solution thereof, the 1/4 diluted solution thereof; and the phosphate buffered saline.

**[Table 4]**

| Concentration | RNA concentration (ng/µL) |
|---|---|
| First example (stock solution) | 35.7 |
| First example (1/2 diluted solution) | 23.9 |
| First example (1/4 diluted solution) | 13.5 |
| Phosphate buffered saline | 15.9 |

According to Fig. 1, it was confirmed that as the concentration of components contained in the respective biological sample preservatives decreases (from the stock solution, to the 1/2 diluted solution and to the 1/4 diluted solution), the preservation state of the preserved biological sample deteriorates, and the solution of the biological sample preservative becomes turbid. In particular, compared with a case in which the biological sample was preserved in the phosphate buffered saline, the same degree of turbidity as that of phosphate buffered saline was generated in the case where the biological sample was preserved in the 1/4 diluted solution of the biological sample preservative of the first example. On the other hand, in the stock solution and the 1/2 diluted solution of the biological sample preservative of the first example, a degree of turbidity was obviously smaller than that in the phosphate buffered saline, and a good preservation state of the biological sample itself could be confirmed. In particular, in the case of the stock solution of the biological sample preservative of the first example, a degree of turbidity was hardly observed, and the biological sample was maintained almost in the same state as that before preservation thereof, and it was confirmed that the biological sample could be very reliably preserved therein even at normal temperature.

Further, according to Table 4, it was confirmed that each concentration of RNA collected from the biological sample after preservation also increased according to the concentration of each component of the biological sample preservative. Specifically, in the case where the biological sample was preserved in the 1/4 diluted solution of the biological sample preservative of the first example, the concentration of RNA extracted from the biological sample was equivalent to the concentration of RNA extracted from the biological sample preserved in the phosphate buffered saline. However, in the case where the biological sample was preserved in the 1/2 diluted solution of the biological sample preservative of the first example, the concentration of RNA extracted from the biological sample showed a distinctly higher value as compared with the concentration of RNA extracted from the biological sample preserved in the phosphate buffered saline. In the case where the biological sample was preserved in the stock solution of the biological sample preservative of the first example, the concentration of RNA extracted from the biological sample showed an extremely high value (twice or more) as compared with the concentration of RNA extracted from the biological sample preserved in the phosphate buffered saline. That is, according to these results, it was confirmed that the preservation effect of the biological sample depends on the concentration of the biological sample preservative of the first example, and that the stock solution and the 1/2 diluted solution of the biological sample preservative of the first example yielded an extremely high preservation effect of the biological sample.

### [Second Experiment]

1 mL of each of the biological sample preservatives of the first to ninth examples was dispensed from the heat-resistant bottle and was dispensed into a corresponding one of 1.5 mL microtubes. Then, 40 mg of a mouse liver was collected as a biological sample, and each of the mouse livers was put into a corresponding one of the microtubes into which the respective biological sample preservatives of the first to ninth examples are dispensed. Thereafter, each of the mouse livers was immersed in a corresponding one of the biological sample preservatives. At this time, 1 mL of phosphate buffered saline as a subject was dispensed into a 1.5 mL microtube, and similarly, 40 mg of a mouse liver was immersed in the phosphate buffered saline. Then, each of the biological samples put into a corresponding one of the microtubes was left to stand and was preserved under normal pressure at 37°C for 24 hours.

After a lapse of 24 hours, each of the livers each serving as a biological sample was taken out from a corresponding one of the microtubes, and RNA extraction was performed on each of the livers. RNA extraction was performed in the same manner as in the first experiment.

Fig. 2 shows an image of each microtube after each of the mouse livers is preserved in a corresponding one of the biological sample preservatives of the first to ninth examples and phosphate buffered saline prepared as a negative control, at 37°C for 24 hours. It is noted that, as a positive control, 40 mg of a mouse liver was collected and then was frozen and preserved at -80°C in a microtube, and the image was photographed in the same manner. Table 5 shows concentrations of RNA respectively obtained from the mouse livers preserved in the respective biological sample preservatives of the first to ninth examples and in the phosphate buffered saline.

**[Table 5]**

| Concentration | RNA concentration (ng/µL) |
|---|---|
| First example | 1206.9 |
| Second example | 1190.5 |
| Third example | 1590.2 |
| Fourth example | 2247.4 |
| Fifth example | 1270.9 |
| Sixth example | 1494.5 |
| Seventh example | 1146.2 |
| Eighth example | 1832.7 |
| Ninth example | 3240.2 |
| Phosphate buffered saline | 387.9 |
| Cryopreserved at -80°C | 2451.9 |

According to Fig. 2, in any one of the biological samples preserved at normal temperature in the respective biological sample preservative solutions of the first to ninth examples, a state equivalent to that of the biological sample preserved at -80°C prepared as a positive control was maintained, and the biological sample preservative solutions were not confirmed to be turbid. On the other hand, the biological sample preserved in phosphate buffered saline prepared as a negative control obviously caused excessive turbidity as compared with the biological samples of the first to ninth examples. That is, it was confirmed that all of the biological sample preservation solutions of the first to ninth examples had an extremely good biological sample preservation effect at normal temperature.

In addition, according to Table 5, each concentration of RNA extracted from a corresponding one of the biological samples preserved at normal temperature in the respective biological sample preservative solutions of the first to ninth examples was measured to be 2.9 times or more higher than the concentration of RNA extracted from the biological sample preserved in the phosphate buffered saline prepared as a negative control. In particular, the concentration of RNA extracted from the biological sample preserved at normal temperature in the biological sample preservative solution of the fourth example was equivalent to the concentration of RNA extracted from the biological sample cryopreserved at -80°C prepared as a positive control, and the concentration of RNA extracted from the biological sample preserved at normal temperature in the biological sample preservative solution of the ninth example was measured to be more than 1.3 times of the concentration of RNA extracted from the cryopreserved biological sample. That is, according to these results, the extremely high preservation effect of the biological sample was confirmed in any one of the biological sample preservatives of the first to ninth examples.

## Claims

1. A biological sample preservative comprising 1.0 to 10.0 mol/L of a substance having a polyatomic monovalent cation represented by a chemical formula NH4⁺ or a compound comprising the polyatomic monovalent cation.

2. The biological sample preservative according to claim 1, wherein a concentration of the substance having the polyatomic monovalent cation or the compound comprising the polyatomic monovalent cation is 1.7 to 5.0 mol/L.

3. The biological sample preservative according to claim 1 or 2, wherein the substance having the polyatomic monovalent cation or the compound comprising the polyatomic monovalent cation contains at least one of ammonium sulfate and ammonium nitrate.

4. The biological sample preservative according to any one of claims 1 to 3, further comprising 0.004 to 10.000 mol/L of a chelating reagent.

5. The biological sample preservative according to claim 4, wherein a concentration of the chelating reagent is 0.006 to 0.500 mol/L.

6. The biological sample preservative according to claim 4 or 5, wherein the chelating reagent contains at least one of sodium citrate and trisodium citrate monohydrate.

7. The biological sample preservative according to any one of claims 1 to 6, further comprising 0.004 to 10.000 mol/L of a buffer.

8. The biological sample preservative according to claim 7, wherein a concentration of the buffer is 0.010 to 5.000 mol/L.

9. The biological sample preservative according to claim 7 or 8, wherein the buffer contains an ethylenediaminetetraacetic acid buffer solution.

10. The biological sample preservative according to any one of claims 1 to 9, further comprising 0.25 to 30 wt% of an excipient.

11. The biological sample preservative according to any one of claims 1 to 10, wherein the biological sample preservative is a freeze-dried powder.

12. A production method of a biological sample preservative, the method comprising a first addition step of adding, to a predetermined solvent, 1.0 to 10.0 mol/L of a substance having a polyatomic monovalent cation represented by a chemical formula NH4⁺ or a compound comprising the polyatomic monovalent cation.

13. The production method of a biological sample preservative according to claim 12, further comprising a second addition step of further adding 0.25 to 30 wt% of an excipient to a solution obtained by the first addition step.

14. The production method of a biological sample preservative according to claim 13, further comprising a freeze-drying step of freeze-drying, at -10°C to -100°C, a solution obtained by the second addition step.

15. The production method of a biological sample preservative according to claim 12, further comprising a dehydration step of dehydrating, under a heated condition or a vacuum condition, a solution obtained by the first addition step.

16. A preservation method of a biological sample comprising a preservation step of immersing, to be preserved, a biological sample in a biological sample preservative solution containing 1.0 to 10.0 mol/L of a substance having a polyatomic monovalent cation represented by a chemical formula NH4⁺ or a compound comprising the polyatomic monovalent cation.

17. A kit for preserving a biological sample, the kit comprising a biological sample preservative containing 1.0 to 10.0 mol/L of a substance having a polyatomic monovalent cation represented by a chemical formula NH4⁺ or a compound comprising the polyatomic monovalent cation.
